(19) Europäisches Patentamt
European Patent Office
Office européen des brevets

(11) Veröffentlichungsnummer : **0 587 552 A1**

# (12) EUROPÄISCHE PATENTANMELDUNG

(21) Anmeldenummer : **93890146.9**

(22) Anmeldetag : **22.07.93**

(51) Int. Cl.[5] : **C10M 133/22,** C10M 175/00, C10L 1/22, C10M 175/02, H01B 3/22, // C10N30/14, C10N40/16

(30) Priorität : **03.08.92 AT 1574/92**

(43) Veröffentlichungstag der Anmeldung : **16.03.94 Patentblatt 94/11**

(84) Benannte Vertragsstaaten : **AT BE CH DE DK ES FR GB GR IE IT LI NL PT SE**

(71) Anmelder : **LOBA FEINCHEMIE AKTIENGESELLSCHAFT**
**Fehrgasse 7**
**A-2401 Fischamend (AT)**

(72) Erfinder : **Meindl, Wolfgang, Dr.**
**Gurkgasse 37**
**A-1140 Wien (AT)**
Erfinder : **Scharrer, Andreas, Dr.**
**Rosenhang 24**
**A-1140 Wieh (AT)**

(74) Vertreter : **Sonn, Helmut et al**
**Patentanwälte Sonn, Pawloy, Weinzinger & Wolfram Riemergasse 14**
**A-1010 Wien (AT)**

(54) **Zusatzstoff für Mineralöle und Mineralölprodukte, speziell elektroisolieröle.**

(57) Mineralöle und Mineralölprodukte, speziell Elektroisolieröle, werden mit einem Zusatzstoff der allgemeinen Formel I

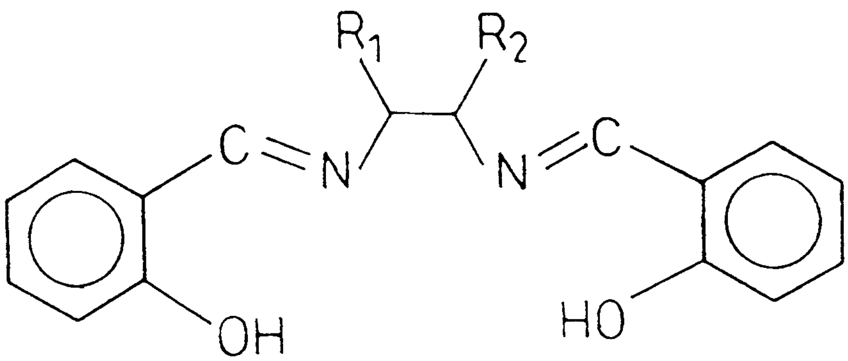

wobei $R_1$ und $R_2$ zusammen mit den Kohlenstoffatomen, an die sie gebunden sind, einen gesättigten fünf- bis siebengliedrigen Kohlenstoff-Ring bilden, der gegebenenfalls mit ein oder mehreren Substituenten, die gleich oder verschieden sein können und ausgewählt sind aus der Gruppe nied.Alkyl und nied.Alkoxy, substituiert ist, versetzt. Dies ermöglicht eine gute Oxydationsbeständigkeit und dadurch eine längere Haltbarkeit der Öle.

Weiters können gebrauchte Mineralöle und Mineralölprodukte unter Verwendung von Verbindungen der Formel I aufbereitet werden, da enthaltene Metallionen gebunden bzw. entfernt werden.

EP 0 587 552 A1

Die vorliegende Erfindung betrifft einen neuen Zusatzstoff für Mineralöle und Mineralölprodukte, speziell Elektroisolieröle.

Mineralöle und Mineralölprodukte bilden sowohl bei ihrer Lagerung als auch bei ihrer Anwendung Oxidationsprodukte oder werden durch Metallionen verunreinigt. Dies führt zu einer deutlichen Verschlechterung der Eigenschaften bzw. zu einer verkürzten Haltbarkeit dieser Substanzen.

Elektroisolieröle sind besonders gereinigte wasserfreie Mineralöle und werden in Vorrichtungen wie Transformatoren, Wandlern, Schaltgeräten und ähnlichen elektrischen Vorrichtungen hoher Leistung angewendet. Diese Vorrichtungen müssen sowohl isoliert als auch gekühlt werden, weil sie gewisse Arbeitstemperaturen nicht überschreiten dürfen.

An diese Öle werden hohe Anforderungen gestellt: Sie müssen gute elektrische (isolatorische) Eigenschaften sowie geringe Viskosität und niedrigen Stockpunkt aufweisen. Vor allem aber müssen sie eine gute Oxidationsbeständigkeit besitzen, weil die Öle nicht nur mit katalytisch wirksamen Metallen, sondern auch mit Luftsauerstoff in Berührung stehen, und das, beispielsweise bei Transformatoren, bei Temperaturen bis zu 90°C.

Gebildete Oxidationsprodukte bewirken eine erhebliche Verschlechterung der elektrischen und der elektroisolatorischen Eigenschaften der Öle und können auch zu einer Schlammbildung führen.

Um die Eigenschaften und die Haltbarkeit von Mineralölen und Mineralölprodukten zu verbessern, werden sie mit Zusatzstoffen vermischt; man spricht dann von "inhibierten, additivierten oder gedopten Ölen". Diese Zusatzstoffe haben die Aufgabe, die Oxidationsbeständigkeit zu erhöhen und eventuell gebildete Ionen zu binden.

Als Zusatzstoffe sind beispielsweise Ionol (2,6-di-tert-butyl-4-Methylphenol), Anthranilsäure, 5-Butylbenzotriazol oder Disalicylidenethylendiamin (DSED) bekannt (z.B. CS-Patentschrift 151 630; Chemical Abstracts Vol.71 (1969), Seite 138, Nr.93268x; Chemical Abstracts Vol.70 (1969), Seite 128, Nr.21662c). Vor allem Ionol und DSED werden großindustriell genutzt, da sie von allen zuvor genannten Stoffen die besten Eigenschaften bei der Anwendung aufweisen. DSED hat jedoch den Nachteil, daß es sich in Mineralölen bzw. Mineralölprodukten nur sehr langsam und in geringer Menge löst und sich Metallkomplexe abscheiden.

Die Erfindung stellt sich daher die Aufgabe, einen Zusatzstoff für Mineralöle und Mineralölprodukte, speziell für Elektroisolieröle, zur Verfügung zu stellen, der die gleiche oder eine verbesserte Stabilisierungswirkung wie DSED aufweist, jedoch dessen Nachteile überwindet.

Der erfindungsgemäße Zusatzstoff ist eine Verbindung der allgemeinen Formel I

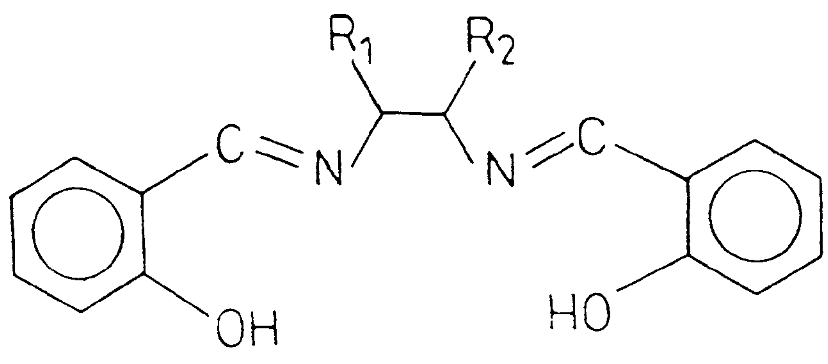

wobei $R_1$ und $R_2$ zusammen mit den Kohlenstoffatomen, an die sie gebunden sind, einen gesättigten fünf- bis siebengliedrigen Kohlenstoff-Ring bilden, der gegebenenfalls mit ein oder mehreren Substituenten, die gleich oder verschieden sein können und ausgewählt sind aus der Gruppe nied.Alkyl und nied.Alkoxy, substituiert ist (nied.Alkyl bzw. nied.Alkoxy bedeuten eine Kettenlänge von 1 bis 4 Kohlenstoff-Atomen).

Besonders bevorzugt ist eine Verbindung der Formel I worin $R_1$ und $R_2$ einen gesättigten, unsubstituierten, sechsgliedrigen Kohlenstoff-Ring bilden: N,N'-Disalicyliden-1,2-cyclohexandiamin (cis/trans).

Die Verbindung der Formel I weist die gleichen bzw. verbesserte Stabilisierungseigenschaften wie DSED auf, hat jedoch bessere Löslichkeitseigenschaften.

Lang haltbare Mineralöle und Mineralölprodukte, speziell Elektroisolieröle, können aufgrund dieser guten Lösungseigenschaften in einfacher Weise durch Vermischung der Basisöle mit dem erfindungsgemäßen Zusatzstoff hergestellt werden.

Die Erfindung betrifft daher auch Mineralöle und Mineralölprodukte, speziell Elektroisolieröle, die eine Verbindung der Formel I, insbesondere N,N'-Disalicyliden-1,2-cyclohexandiamin (cis/trans), als Zusatzstoff enthalten.

Die Erfindung betrifft weiters die Verwendung von Verbindungen der Formel I, insbesondere N,N'-Disalicyliden-1,2-cyclohexandiamin (cis/trans), als Zusatzstoff für Mineralöle und Mineralölprodukte, speziell für

Elektroisolieröle.

Gebrauchte Mineralöle und Mineralölprodukte können durch Anwendung des erfindungsgemäßen Zusatzstoffes in einfacher Weise aufbereitet werden bzw. in ihnen enthaltene Metallionen gebunden bzw. entfernt werden.

Erfindunggemäß wird daher der Zusatzstoff der allgemeinen Formel I, insbesondere N,N'-Disalicyliden-1,2-cyclohexandiamin (cis/trans), auch zur Aufbereitung von gebrauchten Mineralölen und Mineralölprodukten bzw. zur Bindung bzw. Entfernung der in ihnen enthaltenen Metallionen verwendet.

Durch die folgenden Beispiele soll die Erfindung noch näher erläutert werden:

1. Herstellung von N,N'-Disalicyliden-1,2-cyclohexandiamin (cis/trans)

1 Mol 1,2-Cyclohexandiamin (cis/trans, 85 %) und 2 Mol Salicylaldehyd werden in 1 l Lösungsmittel ($C_1$- bis $C_3$-Alkohol, Toluol) 2 bis 4 h unter Rückfluß erhitzt.

Das Reaktionsgemisch wird auf -15°C abgekühlt, das Produkt abgesaugt, mit dem entsprechenden Lösungsmittel gewaschen und bei 50°C im Vakuum getrocknet.

Die Ausbeute betrug 58 bis 62 % bei einem cis/trans-Verhältnis zwischen 22/78 und 25/75.

Das erhaltene Produkt wies einen Schmelzpunkt zwischen 98 und 102°C auf.

2. Untersuchung der Löslichkeit:

Es wurde die Löslichkeit von N,N'-Disalicyliden-1,2-cyclohexandiamin (cis/trans) in handelsüblichem Basisöl untersucht und mit der von DSED verglichen. Während der Lösebeginn von DSED erst bei ca. 85 bis 90°C einsetzt, ist der Lösebeginn von N,N'-Disalicyliden-1,2-cyclohexandiamin (cis/trans) bereits bei ca. 40°C und das Löseende bei ca. 83°C erreicht.

Die Löslichkeit von N,N'-Disalicyliden-1,2-cyclohexandiamin (cis/trans) im Basisöl bei 70°C beträgt etwa 2,3 %. Demgegenüber weist DSED in Basisöl nur eine Löslichkeit von etwa 0,6 bis 0,7 % auf.

3. Vergleich der Stabilisierungseigenschaften von DSED bzw. N,N'-Disalicyliden-1,2-cyclohexandiamin (cis/trans) mittels standardisierter Tests und Vergleich mit den erlaubten Grenzwerten:

Die Tests sind der Fachwelt unter den Bezeichnungen IEC 74, Baader Test (DIN 51554 Teil 1 und 2) und IEC 813 bekannt.

Alle diese Tests dienen der Ermittlung der Oxidationsstabilität von Isolationsflüssigkeiten auf Kohlenwasserstoffbasis unter beschleunigten Bedingungen.

Beim IEC 74-Test wird das zu testende Öl bei 100°C während 164 h unter Durchblasen von Sauerstoff in Anwesenheit eines Kupferdrahtes behandelt.

Anschließend werden die Menge der entstandenen Säuren (ausgedrückt durch die Neutralisationszahl (NZ)) und der Anteil an Schlamm (in Masse-%) in der Probe bestimmt.

Weiters können in diesem Test der dielektrische Verlustfaktor tan $\delta$ und die Grenzflächenspannung (Öl gegen Wasser) bestimmt werden.

Die Alterungsprüfung nach Baader (Baader Test) besteht aus der Behandlung der Ölprobe während 140 h bei 110°C, wobei während dieser Behandlung eine Kupferspirale laufend in das Öl eingetaucht und wieder herausgehoben wird. Danach kann die Verseifungszahl (VZ), der dielektrische Verlustfaktor tan $\delta$, die Menge des gebildeten Schlammes und die Neutralisationszahl bestimmt werden.

Schließlich besteht der IEC 813-Test für Isolieröle in der 164-stündigen Behandlung der Öle unter Durchblasen von Luft bei 120°C in Anwesenheit eines Kupferdrahtes. Anschließend werden wieder Neutralisationszahl, die Menge des geblideten Schlammes, die Grenzflächenspannung und der dielektrische Verlustfaktor tan $\delta$ ermittelt.

Die Ergebnisse der Tests für DSED und N,N'-Disalicylidenethylendiamin sind in Tabelle 1 zusammengefaßt und mit den gültigen Normen verglichen.

Tabelle 1

| | | Grenzwerte lt. Norm | Öl mit 0,01 % DSED | Öl mit 0,01 % erfindungsgemäßem Zusatzstoff |
|---|---|---|---|---|
| **IEC 74** (164 h, $O_2$, 100°C) | | | | |
| NZ | mg KOH/g | ≤ 0,40 | 0,01 | 0,02 |
| Schlamm | Masse-% | ≤ 0,10 | < 0,001 | < 0,001 |
| Grenzfl.sp. | mN/m | - | 37,1 | 38,3 |
| tan δ | $*10^{-3}$ | - | 0,99 | 1,40 |
| **Baader-Test** DIN 51554 Teil 1 u. 2 (140 h, 110°C) | | | | |
| VZ | mg KOH/g | ≤ 0,60 | 0,04 | 0,04 |
| tan δ | $*10^{-3}$ | ≤ 180 | 1,16 | 1,87 |
| Schlamm | Masse-% | ≤ 0,05 | < 0,001 | < 0,001 |
| NZ | mg KOH/g | - | 0,01 | 0,01 |
| **IEC 813** (164 h, Luft, 120°C) | | | | |
| NZ gesamt | mg KOH/g | ≤ 1,5 | 0,04 | 0,05 |
| Schlamm | Masse-% | ≤ 1,0 | < 0,001 | < 0,001 |
| Grenzfl.sp. | mN/m | - | 27,9 | 28,0 |
| tan δ | $*10^{-3}$ | - | 6,41 | 9,22 |

Diese Ergebnisse zeigen deutlich, daß sich der erfindungsgemäße Zusatzstoff in seiner stabilisierenden Wirkung bei diesen Öl-Alterungstests praktisch nicht von DSED unterscheidet und die ermittelten Werte weit unter den geforderten Grenzwerten liegen.

Das isomerenreine trans-Produkt von N,N'-Disalicyliden-1,2-cyclohexandiamin wurde ebenfalls hergestellt. Es zeigte aber in der Anwendung keine weitere Verbesserung gegenüber dem cis/trans-Produkt.

Die angewendete Konzentration des Zusatzstoffes im Elektroisolieröl beträgt im allgemeinen zwischen 2 und 0,001 %, vorzugsweise zwischen 1 und 0,01 %.

## Patentansprüche

1. Zusatzstoff für Mineralöle und Mineralölprodukte, speziell für Elektroisolieröle, der allgemeinen Formel I

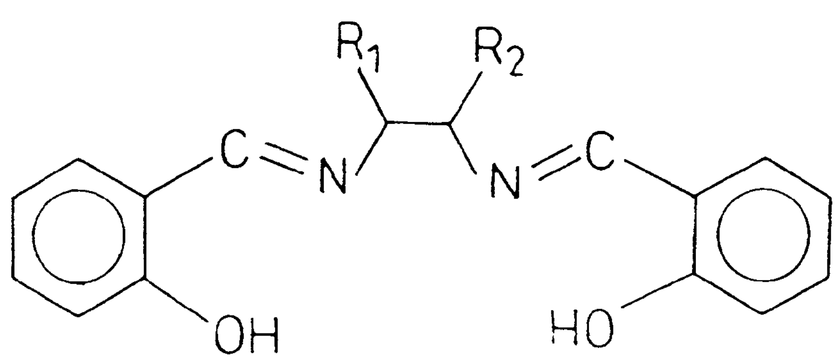

wobei $R_1$ und $R_2$ zusammen mit den Kohlenstoffatomen, an die sie gebunden sind, einen gesättigten fünf- bis siebengliedrigen Kohlenstoff-Ring bilden, der gegebenenfalls mit ein oder mehreren Substituenten, die gleich oder verschieden sein können und ausgewählt sind aus der Gruppe nied.Alkyl und nied.Alkoxy, substituiert ist.

2. Zusatzstoff für Mineralöle und Mineralölprodukte, speziell für Elektroisolieröle, der Formel I, worin $R_1$ und $R_2$ einen gesättigten, unsubstituierten, sechsgliedrigen Kohlenstoff-Ring bilden.

3. Mineralöle und Mineralölprodukte, speziell Elektroisolieröle, dadurch gekennzeichnet, daß sie eine Verbindung der Formel I, insbesondere N,N'-Disalicyliden-1,2-cyclohexandiamin (cis/trans), als Zusatzstoff enthalten.

4. Verwendung von Verbindungen der Formel I, insbesondere N,N'-Disalicyliden-1,2-cyclohexandiamin (cis-/trans), als Zusatzstoff für Mineralöle und Mineralölprodukte, speziell für Elektroisolieröle.

5. Verwendung von Verbindungen der Formel I, insbesondere N,N'-Disalicyliden-1,2-cyclohexandiamin (cis-/trans), zur Aufbereitung von gebrauchten Mineralölen und Mineralölprodukten bzw. zur Bindung bzw. Entfernung der in ihnen enthaltenen Metallionen.

Europäisches Patentamt

# EUROPÄISCHER RECHERCHENBERICHT

Nummer der Anmeldung
EP 93 89 0146

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | US-A-2 282 513 (DOWNING ET AL.)<br>* Seite 4, Spalte 1, Zeile 43 - Zeile 75 *<br>* Tabelle 4 *<br>--- | 1-5 | C10M133/22<br>C10M175/00<br>C10L1/22<br>C10M175/02<br>H01B3/22<br>//C10N30:14, C10N40:16 |
| X | EP-A-0 271 998 (BETZ EUROPE)<br>* das ganze Dokument *<br>--- | 1-5 | |
| X | US-A-3 328 285 (GODAR)<br>* Spalte 6 *<br>* Spalte 13, Zeile 1 - Spalte 14, Zeile 10 *<br>--- | 1-5 | |
| X | GB-A-1 566 106 (NATIONAL RES. DEVEL. CORP.)<br>* das ganze Dokument *<br>--- | 1-5 | |
| X | EP-A-0 476 196 (ETHYL)<br>* Seite 9, Zeile 18; Anspruch 6 *<br>--- | 1-5 | |
| A | GB-A-665 994 (HARRY MILMAN BICKLE)<br>* das ganze Dokument *<br>--- | 5 | RECHERCHIERTE SACHGEBIETE (Int.Cl.5) |
| A | US-A-2 303 546 (GREGER)<br>* das ganze Dokument *<br>----- | 5 | C10M<br>H01B<br>C10L |

Der vorliegende Recherchenbericht wurde für alle Patentansprüche erstellt

| Recherchenort | Abschlußdatum der Recherche | Prüfer |
|---|---|---|
| DEN HAAG | 15. Dezember 1993 | De La Morinerie, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)